# FASCICULE DE BREVET EUROPEEN

(11) **EP 1 260 597 B1**
(45) Date de publication et mention de la délivrance du brevet: **25.02.2009**
(21) Numéro de dépôt: 02018001.4
(22) Date de dépôt: 17.06.1999
(51) Int. Cl.: C12Q 1/68

(54) **Procédé de marquage d'un acide ribonucléique avec purification**
Verfahren zur Markierung einer Ribonukleinsäure, mit Reinigung
Process for labeling a nucleic acid, including purification

(30) Priorité: 17.06.1998 FR 9807870
(43) Date de publication de la demande: 27.11.2002
(62) Demande divisionnaire de: 99957062.5
(73) Titulaire: BIO MERIEUX, 69280 Marcy l'Etoile (FR)
(72) Inventeur: Laayoun, Ali, 69007 Lyon (FR)
(74) Mandataire: Guerre, Dominique

(56) Documents cités:
- WO-A-88/04300
- WO-A-93/16094
- US-A- 5 317 098
- US-A- 5 348 855
- US-A- 5 688 670
- TUNG C.-H.; WEI Z.; LEIBOWITZ M.J.; STEIN S.: 'Design of peptide-acridine mimics of ribonuclease activity' PROCEEDINGS OF THE NATIONAL ACADEMY OF SCIENCES OF THE UNITED STATES OF AMERICA vol. 89, no. 15, 1992, pages 7114 - 7118, XP002252419
- BRESLOW R.; XU R.: 'Recognition and catalysis in nucleic acid chemistry' PROCEEDINGS OF THE NATIONAL ACADEMY OF SCIENCES OF THE UNITED STATES OF AMERICA vol. 90, no. 4, 1993, pages 1201 - 1207, XP002252420
- MODAK A.S. ET AL: 'Toward chemical ribonucleases 2. Synthesis and characterization of nucleoside-bipyridine conjugates. Hydrolytic cleavage of RNA by their copper(II) complexes' JOURNAL OF THE AMERICAN CHEMICAL SOCIETY vol. 113, no. 1, 1991, pages 283 - 291, XP002252421
- YOSHINARI K.; YAMAZAKI K.; KOMIYAMA M.: 'Oligamines as simple and efficient catalysts for RNA hydrolysis' JOURNAL OF THE AMERICAN CHEMICAL SOCIETY vol. 113, no. 15, 1991, pages 5899 - 5901, XP002252422
- FURUTA T. ET AL: 'Direct esterification of phosphates with various halides and its application to synthesis of cAMP Alkyl triesters' JOURNAL OF THE CHEMICAL SOCIETY, PERKIN TRANSACTIONS 1 1993, pages 3139 - 3142, XP002252423
- ROSENBERG M.: 'Isolation and sequence determination of the 3'-terminal regions of isotopically labelled RNA molecules.' NUCLEIC ACIDS RESEARCH vol. 1, no. 5, Mai 1974, ENGLAND, pages 653 - 671, XP009043792

## Description

La présente invention concerne un nouveau procédé de marquage d'un acide ribonucléique (ARN) de synthèse ou naturel.

Par ARN de synthèse, il faut comprendre ARN obtenu par une technique mise au point par l'homme, par exemple une technique d'amplification (PCR suivi d'une transcription) ou d'amplification transcriptionnelle (TMA). Par ARN naturel, il faut comprendre ARN obtenu par extraction d'une cellule, par exemple ARN messager, ribosomal, de transfert.

L'état de la technique montre que de nombreuses méthodes existent pour marquer des nucléotides, des oligonucléotides ou des acides nucléiques ; les oligonucléotides et les acides nucléiques seront désignés par le terme polynucléotides. En ce qui concerne les oligonucléotides, le marquage peut s'effectuer soit lors de la synthèse, soit par incorporation d'au moins un nucléotide marqué.

Une première méthode consiste à fixer le marqueur sur la base, que celle-ci soit naturelle ou modifiée. Une deuxième méthode propose de fixer le marqueur sur le sucre, là encore qu'il soit naturel ou modifié. Une troisième méthode a pour objet la fixation du marqueur sur le phosphate.

La marquage sur la base a été notamment utilisé dans l'approche de marquage des acides nucléiques par incorporation de nucléotides directement marqués.

Le marquage sur le sucre est souvent utilisé dans le cas des sondes nucléiques préparées par synthèse chimique.

Le marquage sur le phosphate a été aussi utilisé pour introduire des bras fonctionnalisés et des marqueurs lors de la synthèse chimique des polynucléotides.

En fait l'homme du métier, qui doit effectuer un marquage d'un nucléotide, ou d'un analogue de nucléotide ou d'un acide nucléique, est enclin à effectuer cette fixation sur la base ou sur le sucre qui lui offrent plus de commodité et d'alternative. C'est d'ailleurs ce qui ressort de l'étude de nombreux documents, tels que EP-A-0.329.198, EP-A-0.302.175, EP-A-0.097.373, EP-A-0.063.879, US-A-5,449,767, US-A-5,328,824, WO-A-93/16O94, DE-A-3.910.151, EP-A-0.567.841 pour la base ou EP-A-0.286.898 pour le sucre.

Néanmoins, ces techniques ont certains inconvénients, dont les deux principaux sont la gêne stérique et l'influence engendrées par la présence d'un marqueur.

Dans le cas d'un marquage effectué sur la base, la gêne stérique est due à l'empiétement du marqueur sur l'espace où est présente une base voisine, que cette base voisine soit portée par un nucléotide adjacent du même brin ou par le brin complémentaire. Il est assez évident également que la présence du marqueur sur la base peut gêner l'efficacité et la spécificité, lors de l'incorporation enzymatique, et peut influencer la qualité des liaisons hydrogènes entre les deux brins complémentaires, ce qui peut nuire à l'hybridation.

Dans le cas d'un marquage sur le sucre, la gêne stérique est due à l'empiétement du marqueur sur l'espace où est présent un sucre adjacent porté par le même brin. Cette présence du marqueur peut éloigner deux bases adjacentes portées par le même brin et empêcher, par la suite, la bonne hybridation avec le brin complémentaire, du fait que les liaisons hydrogènes ne sont pas optimales entre les brins.

La fixation du marqueur sur le phosphate est une technique plus complexe que la technique consistant à fonctionnaliser la base ou le sucre.

Pourtant dans certains documents, ont été proposées des techniques de marquage du phosphate. C'est par exemple le cas avec le document EP-A-0.285.058, qui décrit le marquage d'un nucléotide par fixation du marqueur sur le phosphate, ce dernier étant fixé sur le sucre en position 2' et/ou 5' quand le nucléotide est un désoxyribonucléotide, et en position 2', 3' et/ou 5' quand le nucléotide est un ribonucléotide. Il décrit également un polynucléotide ou oligonucléotide qui comprend au moins un nucléotide marqué comme décrit ci-dessus ; ce nucléotide est incorporé dans le polynucléotide ou l'oligonucléotide lors de la synthèse.

Toutefois, le marquage, proposé par le document EP-A-0.285.058, ne permet pas d'obtenir un marquage uniforme des acides nucléiques. En effet, l'incorporation des nucléotides marqués dans les polynucléotides ne peut pas être contrôlée, il dépend entièrement de la composition en polynucléotides qui sera synthétisée. Ainsi certains polynucléotides peuvent contenir de nombreux nucléotides marqués alors que d'autres peuvent ne pas en contenir du tout. L'intensité du signal émis par ces acides nucléiques ne sera donc pas uniforme, ce qui risque de rendre l'interprétation des résultats difficile lors de leur détection.

Dans ce cas, le marquage est un marquage biologique par incorporation, où l'on ne contrôle pas la position des nucléotides marqués.

Le document US-5,573,913 décrit le marquage d'un acide ribonucléique, selon lequel on incorpore dans ledit acide des désoxynucléotides porteurs d'un marqueur non radioactif, l'un au moins de ces désoxynucléotides étant fixé sur l'extrémité 3' par action de la transférase terminale.

Le document US-A-5,317.098 concerne des acides nucléiques qui sont marqués à leur extrémité 5'. Cette fixation, utilise l'imidazole et un bras de liaison. Il n'y a pas de fragmentation associée. De plus, le phosphate est rapporté, ily a donc utilisation de kinase.

Le document WO-A-96/28460 décrit le marquage d'acides nucléiques, notamment d'ARN, par incorporation à l'extrémité de fragments d'ARN, de dérivés de nucléosides qui servent de substrat à la RNA polymérase.

Néanmoins et logiquement, il y aura présence d'un phosphate à chaque extrémité libre de l'acide nucléique, ce qui induit au moins une étape supplémentaire. Ce marquage n'est pas associé à une fragmentation.

De plus, en ce qui concerne les documents précédents, le marquage est réalisé sur des acides nucléiques de grandes tailles. En effet, aucune phase de fragmentation, également appelée phase de clivage, n'a été décrite avant les étapes de marquage. Ainsi, lorsque l'on hybride ces acides nucléiques cibles sur des sondes de capture, les duplex formés après hybridation ne sont pas stables. C'est également le cas lorsque les polynucléotides sont utilisés en tant que sondes de détection. Les raisons peuvent être dues à la gêne stérique ou au manque de spécificité entre le polynucléotide, qui a été synthétisé, et sa cible qui n'est pas forcément de même taille. Il va donc y avoir une perte quantitative et qualitative du signal.

La gêne stérique peut être le fait, non seulement, de la longueur de l'acide nucléique, mais également, de l'existence ou de la conservation de structures secondaires. La fragmentation, permet de détruire ces structures et ainsi d'optimiser l'hybridation. Cette gêne stérique joue un rôle particulièrement important dans le cas de l'hybridation sur des surfaces contenant des sondes de capture à forte densité, par exemple les puces à ADN mises au point par la société Affymetrix ("Accessing Genetic Information with Iligh-Density DNA arrays", M. Shee et al., Science, 274, 610-614. "Light-generated oligonucleotide arrays for rapide DNA sequence analysis", A. Caviani Pease et al., Proc. Natl. Acad. Sci. USA, 1994, 91, 5022-5026). Dans cette technologie, les sondes de capture sont généralement de tailles réduites, autour de vingt nucléotide.

En ce qui concerne la fragmentation des acides nucléiques, de nombreuses méthodes sont décrites dans l'état de la technique.

Premièrement, la fragmentation peut être enzymatique, c'est-à-dire que la fragmentation acides nucléiques peut être réalisée par des nucléases (DNases ou RNases). On génère alors des fragments de petites tailles avec des extrémité 3'-OH, 5'-OH, 3'-phosphate, 5'-phosphate. Selon Tung et al., PNAS USA (1992) 89, 7114-7118, on décrit un tripeptide mimant le site actif de la RNase. M. Rosenberg, Nucleic Acids Research, vol. 1. N°5 May 1974. pages 653-670, concerne l'obtention de fragments de l'extrémité 3' d'ARN, obtenus par fragmentation enzymatique d'un acide ribonucléique radio marqué.

Deuxièmement, la fragmentation peut être chimique. Par exemple dans le cas des ADN, on peut effectuer la dépurination ou la dépyrimidination des ADN, qui sont alors fragmentés en présence d'une base par un mécanisme dit de β-élimination ". La fragmentation des ADN peut être réalisée par des mécanismes d'oxydation, d'alkylation, d'addition de radicaux libres entre autres. Pour fragmenter les ARN, on utilise des cations métalliques souvent associés à des molécules organiques utilisées comme catalyseurs chimiques, par exemple l'imidazole. Cette fragmentation est préférentiellement réalisée en milieu alcalin et génère des fragments avec des extrémités 3'-phosphate. Ainsi, selon R. Breslow et al., PNAS USA (1993) 90, 1201-1207, on décrit la fragmentation d'ARN par des tampons imidazole selon Modak et al.. J. Am. Chem. Soc. (1991) 113, 283-291, on décrit la fragmentation d'ARN par des complexes au cuivre (11) de conjuguées nucléoside-bipyridine qui agissent par hydrolyse des liaisons phosphodiester; selon Yoshinari et al., J. Am. Chem. Soc. (1991) 113, 5899-5901, on met en évidence l'activité hydrolytique d'oligoamines sur de ARN.

Cependant, ces fragmentations n'ont pas pour objet de faciliter ou de permettre le marquage.

Le document WO-A-88/04300 propose une méthode qui permet la fragmentation et le marquage d'ARN, la fragmentation s'effectuant par l'intermédiaire d'ARN ayant des propriétés enzymatiques, les ribozymes. Cette fragmentation par les ribozymes libère pour chaque coupure une extrémité (5') HO-acide nucléique et une extrémité (3') HO-PO₂-acide nucléique. Le marquage, qui est uniquement radioactif, s'effectue ensuite par l'intermédiaire d'une enzyme d'incorporation (kinase) qui permet l'incorporation d'un phosphate radioactif rapporté, provenant d'une molécule de γ-GTP. Cette fixation s'effectue uniquement au niveau de l'extrémité 5', De plus, la fragmentation est réalisée uniquement par des ribozymes, ce qui implique qu'il y ait une spécificité entre ceux-ci et les acides nucléiques cibles à couper. Le phosphate fait alors office de marqueur.

Notre invention permet une fixation d'un marqueur au niveau du seul phosphate, d'un fragment d'acide nucléique, libéré lors de la coupure. Il n'y a aucune spécificité, la fragmentation pouvant être réalisée sur n'importe quel type d'acide nucléique et ce de manière aléatoire. En fait, notre procédé permet par exemple la préparation de sonde de détection. Enfin, le phosphate n'est qu'un bras de liaison entre l'acide nucléique et le marqueur.

Aucun procédé de fragmentation avant marquage en une ou deux étapes n'a été décrit dans l'art antérieur.

La présente invention propose donc un procédé qui pallie les inconvénients précédemment cités. En effet, il permet d'obtenir un marquage uniforme des fragments d'ARN, dès la fragmentation terminée. De plus, la fragmentation permet d'obtenir des fragments d'une taille optimale pour une éventuelle hybridation. L'hybridation étant de meilleure qualité, la révélation de cette hybridation sera plus rapide et efficace.

Par marquage, on entend la fixation d'un marqueur capable de générer directement ou indirectement un signal détectable. Une liste non limitative de ces marqueurs suit :
- les enzymes qui produisent un signal détectable par exemple par colorimétrie, fluorescence, luminescence, comme la péroxydase de raifort, la phosphatase alcaline, la α-galactosidase, la glucose-6-phosphate déshydrogénase,
- les chromophores comme les composés fluorescents, luminescents, colorants,
- les groupements à densité électronique détectable par microscopie électronique ou par leur propriété électrique comme la conductivité, l'ampérométrie, la voltamétrie, l'impédance,
- les groupements détectables, par exemple dont les molécules sont de tailles suffisantes pour induire des modifications détectables de leurs caractéristiques physiques et/ou chimiques, cette détection peut être réalisée par des méthodes optiques comme la diffraction, la résonance plasmon de surface, la variation de surface, la variation d'angle de contact ou des méthodes physiques comme la spectroscopie de force atomique, l'effet tunnel,
- les molécules radioactives comme le ³²P, le ³⁵S ou le ¹²⁵I.

Des systèmes indirects peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte l'agent de liaison. L'anti-ligand peut être détectable directement par les marqueurs décrits au paragraphe précédent ou être lui-même détectable par un ligand/anti-ligand.

Ces systèmes de détection indirects peuvent conduire, dans certaines conditions, à une amplification du signal. Cette technique d'amplification du signal est bien connue de l'homme du métier, et l'on pourra se reporter aux demandes de brevet antérieures FR-2 781 802 (FR98/10084) ou WO-A-95/08000 de la demanderesse ou à l'article J. Histochem. Cytochem. 45 : 481-491, 1997.

A cet effet, la présente invention concerne un procédé de marquage d'un acide ribonucléique (ARN) de synthèse ou naturel, caractérisé en ce qu'il consiste :
- à fragmenter l'ARN, et
- à marquer au niveau du phosphate terminal situé à l'extrémité 3' de chaque fragment dudit ARN, ledit phosphate terminal ayant été libéré lors de la fragmentation.

Selon un mode préférentiel de fonctionnement, le marquage de l'extrémité 3' de chaque fragment de l'ARN s'effectue pour chaque fragment à l'exception du fragment constituant l'extrémité 3' de l'ARN de départ.

Selon un premier mode de réalisation, la fragmentation et le marquage s'effectue en une étape.

Selon un second mode de réalisation, la fragmentation et le marquage s'effectue en deux étapes.

Quel que soit le mode de réalisation, le marquage de l'extrémité 3' d'un fragment d'ARN s'effectue par fixation, sur le phosphate en position 2', en position 3' ou en position 2'-3'-monophosphate cyclique, par rapport au ribose, d'une fonction réactive portée par un marqueur ou pouvant s'associer ultérieurement à au moins un marqueur. Lorsqu'il y a au moins deux marqueurs, il s'agit d'une technique d'amplification du signal.

La fragmentation et/ou le marquage de l'extrémité 3' d'un fragment d'ARN s'effectue par fixation, sur le phosphate en position 2', en position 3' ou en position 2'-3'-monophosphate cyclique, par rapport au ribose, d'une fonction nucléophile, électrophile, halogénure portée par un marqueur ou pouvant s'associer ultérieurement à un marqueur.

La fragmentation de l'ARN s'effectue par voie chimique.

Les voies enzymatique et physique qui ne sont pas couvertes par l'invention sont décrites.

La fragmentation par voie enzymatique de l'ARN est réalisée par des nucléases.

La fragmentation par voie chimique de l'A RN est réalisée par des cations métalliques associés ou non à un catalyseur chimique.

Dans ce cas, les cations métalliques sont des ions Mg⁺⁺, Mn⁺⁺, Cu⁺⁺, Co⁺⁺ et/ou Zn⁺⁺, et le catalyseur chimique est constitué par de l'imidazole, un analogue substitué, par exemple le N-méthyl-imidazole, ou toute molécule chimique ayant une affinité pour l'ARN et portant un noyau imidazole ou un analogue substitué.

La fragmentation par voie physique de l'ARN est réalisée par sonication ou par radiation.

Dans tous les cas de figure, le marquage de l'extrémité 3' d'un fragment d'ARN s'effectue par fixation, sur le phosphate relié à la position 2', à la position 3' ou à la position 2'-3'-monophosphate cyclique du ribose, d'une molécule R-X, où R est constitué par le marqueur et X est l'agent de liaison entre le marqueur et l'ARN, tel qu'un groupement hydroxyle, amine, hydrazine, alcoxylamine, halogénure d'alkyle, phényl-méthyle halogénure, iodoacétamide, maléimide.

La présente description concerne également un fragment d'ARN obtenu par le procédé, selon les caractéristiques ci-dessus exposées, ledit fragment d'ARN comportant, d'une part, un seul nucléotide marqué au niveau du phosphate terminal situé à l'extrémité 3' du fragment d'ARN, ledit phosphate terminal ayant été libéré lors de la fragmentation, et d'autre part, au moins un autre nucléotide dont la base (purique : adénine/guanine ou pyrimidique : uracyle/cytosine) est identique à celle du nucléotide marqué.

Ce fragment d'ARN comporte de 10 à 100 nucléotides, préférentiellement de 30 à 70 et préférentiellement de 40 à 60 nucléotides. Le fragment d'ARN peut comporter au moins un nucléotide thiophosphate.

De plus, le nucléotide marqué est un nucléotide thiophosphate.

La description concerne l'utilisation d'un fragment d'ARN, tel que défini ci-dessus, comme sonde de détection d'un ARN et/ou d'un ADN ou d'un fragment d'ARN et/ou d'ADN.

La description concerne enfin l'utilisation d'un fragment d'ARN, tel que défini ci-dessus, comme cible marqué pouvant se fixer sur une sonde de capture.

Les figures ci-jointes montrent différents modes de synthèse de fragments d'ARN marqués selon l'invention, ainsi que les fragments ainsi obtenus. Ils représentent des modes particuliers de réalisation et ne peuvent pas être considérés comme limitant la portée de la présente invention.

La figure 1 représente une vue schématique de la fragmentation, chimique d'un ARN, en présence de cations Mn⁺⁺ et d'imidazole.

La figure 2 représente une vue schématique de la fragmentation et du marquage d'un ARN, par un marqueur portant une fonction nucléophile.

La figure 3 représente un marqueur utilisable avec le procédé exposé à la figure 2, ce marqueur étant constitué par de la fluorescéine-cadavérine.

La figure 4 représente un marqueur utilisable avec le procédé exposé à la figure 2, ce marqueur étant constitué par de la fluorescéine-hydrazide.

La figure 5 représente un marqueur halogéné utilisable avec le procédé selon l'invention.

La figure 6 représente une vue schématique de l'extrémité 3' d'un fragment d'ARN obtenu par un procédé selon la présente invention, où le marqueur est fixé sur un phosphate naturel, Z étant un bras de couplage, également appelé bras espaceur, tel que défini dans une précédente demande de brevet de la demanderesse déposée le 1^{er} août 1997 et publiée sous le numéro WO-A-98/05766 (PCT/FR97/01445). Cette définition de Z est également vraie pour les figures 7 et 8.

La figure 7 représente une vue schématique de l'extrémité 3' d'un fragment d'ARN obtenu par un procédé selon la présente invention, où le marqueur est fixé sur un thiophosphate.

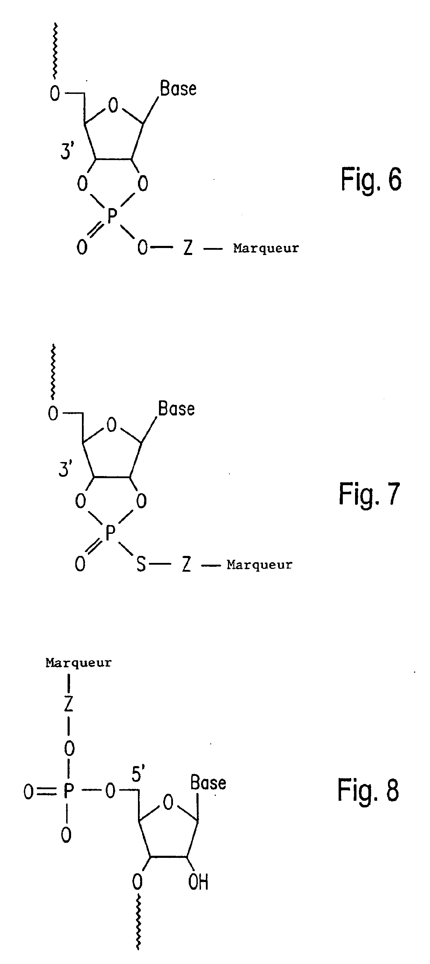

Enfin, la figure 8 représente une vue schématique de l'extrémité 5' d'un fragment d'ARN obtenu par un procédé selon la présente invention, où le marqueur est fixé sur un phosphate naturel.

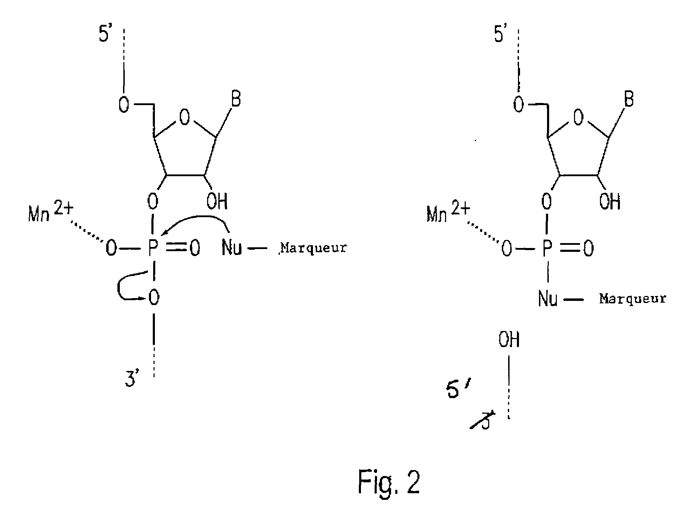

Le procédé décrit sur les figures peut être en une étape, comme c'est le cas sur la figure 2 où la fragmentation et le marquage sont réalisés conjointement par un nucléophile marqué.

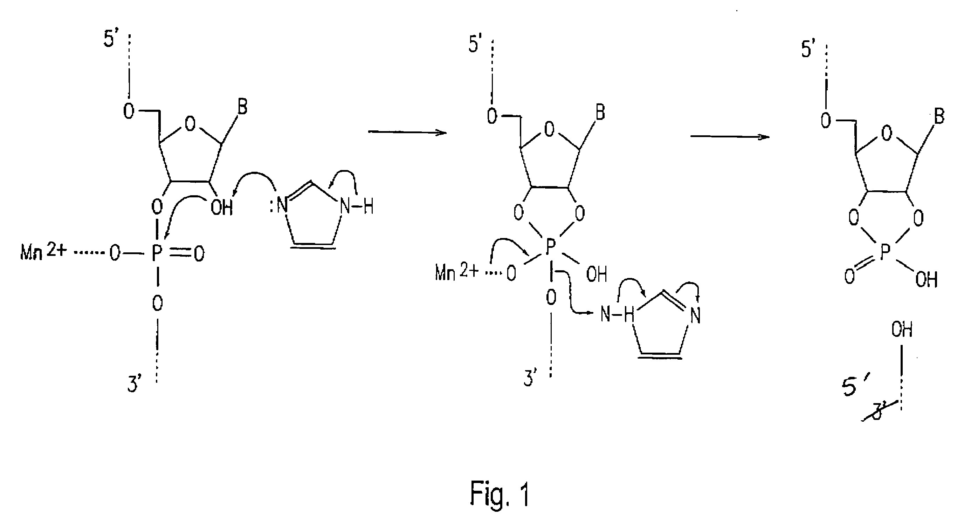

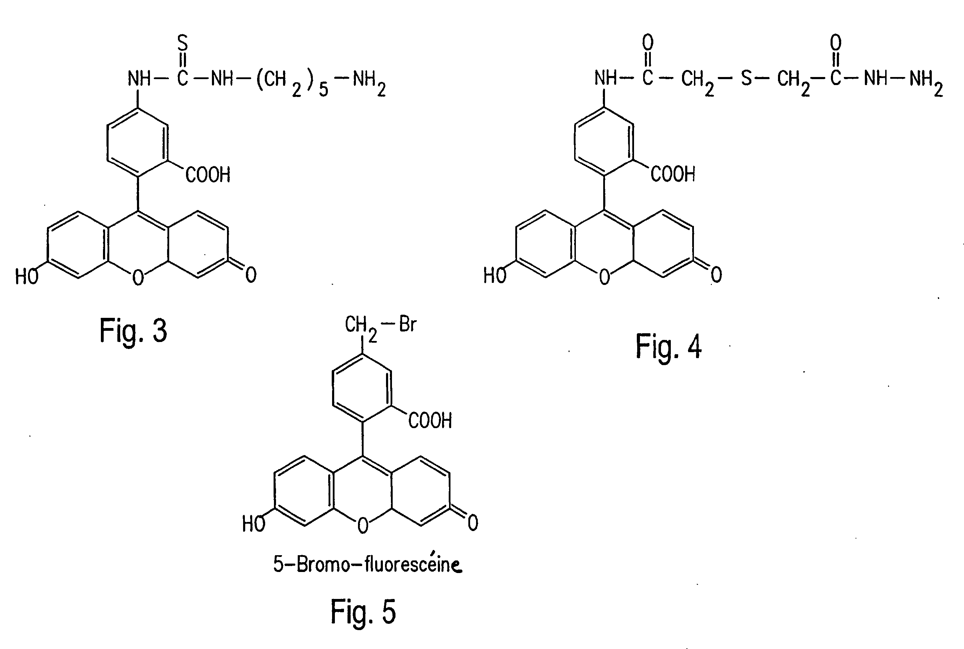

Le procédé peut être réalisé également en deux étapes. Une première étape de fragmentation selon la figure 1, par action de l'imidazole par exemple. Enfin, une seconde étape de marquage par l'intermédiaire d'un marqueur, représenté de manière non limitative aux figures 3 à 5 ; un tel marquage s'effectue sur le phosphate libéré et est exposé aux figures 6 à 8.

### Exemple 1 - Préparation des ARN amplicons et ARN transcrits

### A - Préparation des amplicons naturels :

Les amplicons naturels (ARN simple brin) sont préparés en utilisant la technique d'amplification TMA (Transcription-Mediated Amplification) développée par Gen Probe (San Diego, CA). Le brin d'ARN amplifié correspond à la séquence 16S des ribosomes de *Mycobacterium tuberculosis* (ATCC -27294). L'ARN 16 S a été cloné dans un plasmide et transformé dans des bactéries issues du kit TA Cloning Dual Promoter (Réf. : K2050-01- Invitrogen, Groningen, Pays Bas). Après culture en milieu LB (décrit par exemple dans le Maniatis) et extraction de l'ADN bactérien par lyse alcaline, le brin a été transcrit par le kit "Ampli Scribe T7 Transcription" (Réf. : AS 2607 - Epicentre Technologies (Madison, WI)). Le nombre de copies par microlitre a été determiné par mesure de l'absorbance à 260 nm.

### B - Préparation des ARN transcrits à partir d'ADN obtenu par TMA :

Il convient tout d'abord de noter que le produit d'amplification TMA contient, en plus des ARN, des amplicons ADN, dans une proportion de sensiblement 90 % d'ARN pour 10 % d'ADN. Ce sont ces amplicons ADN qui ont été aussi utilisés pour produire des ARN simple brin par transcription *in vitro.*

On a tout d'abord dans une première étape obtenu des amplicons avec le kit Mycobacterium Tuberculosis Direct (MTD) de Gen Probe (Réf. : 1001E), de la même manière que précédemment selon le protocole décrit dans l'étape 1-A ci-dessus.

Dans une seconde étape, la transcription a été réalisée sur une matrice ADN obtenue par TMA de la première étape, à partir d'une matrice ARN 16S de *Mycobacterium tuberculosis.*

La transcription a été réalisée à partir de 5 µl de produits TMA en utilisant le kit MEGAscript T7 (AMBION, Austin, TX, Ref :1334). La réaction a eu lieu pendant une heure à 37°C.

### C - Préparation des ARN transcrits à partir d'ADN obtenus par PCR :

A partir d'un isolat bactérien (ATCC-27294), cultivé sur milieu Lowenstein-Jensen, une ou deux colonies (3-5 mm de diamètre équivalent à 10⁸ bactéries) ont été récoltées à l'aide d'une spatule et remis en suspension dans 250 µl d'eau stérile dans un tube Eppendorf de 1,5 ml. Les acides nucléiques ont été extraits du matériel cellulaire de la suspension bactérienne par une agitation vigoureuse au moyen d'un vortex en présence de billes de verre. Une telle extraction est bien décrite dans les demandes de brevet FR-2 768 743 (FR97/12164) du 23 septembre 1997 et FR-2 781 500 (FR98/09583) du 23 juillet 1998 déposées par la demanderesse.

Un aliquote de 5 µl du lysat a été ajouté directement à la réaction de PCR. Il est aussi possible d'ajouter 20 ng d'ADN plasmidique directement à la réaction de PCR.

La région 16S hypervariable a été amplifiée par PCR en utilisant des amorces spécifiques du genre *Mycobacterium* (positions 213-236 et 394-415 sur la séquence de référence de *M. tuberculosis,* M20940, Genbank), la taille de l'amplicon étant de 202 paires de bases (pb). Les amorces contiennent aussi des séquences de promoteurs de bactériophage T3 ou T7, à leur extrémité 5'. Dans la représentation des amorces qui suit, la séquence des promoteurs est en caractères minuscules et gras, alors que la séquence des mycobactéries est en majuscules :
T3 - M1 5'-**aattaaccctcactaaaggg**AACACGTGGGTGATCTGCCCTGCA
T7 - M2 5'-**gtaatacgactcactatagggc**TGTGGCCGGACACCCTCTCA

La PCR a été réalisée dans un volume réactionnel de 100 µl contenant 50 mM KCl, 10 mM de Tris, pH = 8,3, 1,5 mM de MgCl₂, 0,001% (m/v) de gélatine, 5 % (v/v) de DMSO, 0,5 µM de chaque amorce, 200 µM de chacun des quatre désoxynucléotides triphosphates, et 1,5 unités de polymérase Taq (AmpliTaq, Perkin-Elmer, Norwalk, CT). La réaction de PCR a été pratiquée dans un thermocycleur 2400 Perkin-Elmer (Norwalk, CT) avec une étape initiale de dénaturation à 94°C de 5 minutes (mn), et 35 cycles de 45 secondes (s) à 94°C, 30 s à 60°C, 30 s à 72°C, suivis de 10 mn à 72°C après le dernier cycle. Les produits de la réaction de PCR ont été analysés par électrophorèse sur gel d'agarose.

Les amplicons contenant les promoteurs ont été utilisés pour produire des ARN simple brin par transcription *in vitro.* Chaque réaction d'un volume de 20 µl contient approximativement 50 ng de produits PCR, 20 unités de polymérase T3 ou T7 (Promega, Madison, WI)), 40 mM de tampon Tris-Acétate, pH = 8,1, 100 mM d'acétate de Magnésium [Mg(AcO)₂], 10 mM de DTT, 1,25 mM de chaque nucléotide triphosphate (ATP, CTP, GTP, UTP). La réaction a eu lieu pendant une heure à 37°C.

### D - Préparation des ARN transcrits contenant des thiophosphates à partir d'ADN obtenus par PCR :

Les amplicons obtenu par PCR, selon l'étape précédente 1-C, contenant les promoteurs ont été utilisés pour produire des ARN simple brin contenant des thiophosphates par transcription *in vitro.* Chaque réaction d'un volume de 20 µl contient alors approximativement 50 ng de produits PCR, 20 unités de polymérase T3 ou T7 (Promega, Madison, WI)), 40 mM de tampon Tris-Acétate, pH = 8,1, 100 mM d'acétate de Magnésium [Mg(AcO)₂], 10 mM de DTT, 1,25 mM de chaque sorte de nucléotides triphosphates ou thiophosphates (ATP-α-thiophosphate (ATP-α-S) ou CTP-α-thiophosphate (CTP-α-S)) selon deux solutions différentes :
- ATP-α-S, CTP, GTP et UTP, ou
- ATP, CTP-α-S, GTP et UTP
La réaction a eu lieu pendant une heure à 37°C.

Le nucléotide thiophosphate utilisé était présent à 100% des 1,25 mM, venant donc en substitution du nucléotide naturel correspondant. Dans chaque cas, le produit de transcription a été analysé par électrophorèse sur gel de polyacrylamide à 6% en présence d'urée 7M. Après coloration au bromure d'éthidium, la taille du transcrit est contrôlée et quantifiée par rapport à un standard déposé sur le gel.

Les nucléotides ATP-α-S et CTP-α-S ont été obtenus de N&N Life Science Products (Boston, MA - USA).

### Exemple 2 - Fragmentation chimique des ARN :

La fragmentation chimique des ARN est souvent catalysée par des cations métalliques (Mn⁺⁺, Mg⁺⁺ ...) qui, en se liant au groupement phosphate, neutralisent la charge négative de l'oxygène et facilite ainsi l'attaque nucléophile sur le phosphate par l'hydroxyle en position 2' du ribose.

Cette attaque nucléophile peut être renforcée par la présence de molécules qui sont donneurs et accepteurs de protons, telles que le noyau imidazole (R. Breslow et R. Xu, Proc. Natl. Acad. Sci. USA, 90, 1201-1207, 1993), comme cela est bien représenté dans la figure 1.

La fragmentation peut être réalisée à différentes températures et dans différentes conditions. Selon le descriptif qui va suivre, deux types de fragmentations différentes ont été réalisées.

Il peut y avoir une fragmentation à 60°C. Dans ce cas, les transcrits ARN 16S de *Mycobacterium tuberculosis*, ci-après appelée *Mycobactéries*, qui comportent 330 nucléotides (environ 66 pmoles), sont incubés dans une solution aqueuse d'imidazole (30mM) et de chlorure de manganèse (30mM) à 60°C pendant 30 min. Le volume total de la solution de fragmentation est de 100 µl. L'ARN fragmenté est ensuite analysé sur gel de polyacrylamide (6X, urée 7M) et coloré au bromure d'éthidium.

Il peut également y avoir une fragmentation à 95°C. Alors, les transcrits ARN 16S de 330 nucléotides (66 pmoles) *Mycobactéries* sont incubés dans une solution aqueuse de chlorure de magnésium (30mM) à 95°C pendant 30 min. Le volume total de la solution de fragmentation est de 100 µl. L'ARN fragmenté est ensuite analysé sur gel de polyacrylamide (6X, urée 7M) et coloré au bromure d'éthidium.

Dans les deux types de fragmentation, l'analyse sur gel montre la disparition du produit de départ et l'apparition de plusieurs fragments plus courts, dont la population la plus importante a une taille comprise entre 20et 50 nucléotides.

Ce sont ces deux protocoles qui ont été utilisés pour introduire un marqueur fluorescent sur la chaîne d'ARN pendant sa fragmentation.

### Exemple 3 - Marquage pendant la fragmentation par introduction de marqueurs portant une fonction nucléophile :

Une fonction plus nucléophile que l'hydroxyle, en position 2' du ribose, peut attaquer le phosphate neutralisé et permettre ainsi la fragmentation de la chaîne d'ARN en générant des fragments liés à cette fonction, via le groupement phosphate. Selon la figure 2, cette fonction peut être liée à un marqueur pour générer des fragments d'ARN marqués.

La fluorescéine-cadavérine, exposée en figure 3, portant une fonction amine et la fluorescéine-hydrazide, détaillée en figure 4, portant une fonction hydrazide ont été utilisées pour marquer un ARN 16S (330 nucléotides) de *Mycobactéries*, pendant la fragmentation à 65°C. De tels ARN ont été obtenus selon l'exemple 1B

La fluorescéine cadavérine et la fluorescéine hydrazide ont été solubilisées dans de la DMF à une concentration finale de 7,5 mM. Elles proviennent de chez Molecular Probes (Eugene, OR, USA).

A la cible d'ARN 16S (66 pmoles) en solution dans le tampon de fragmentation à 65°C (exemple 2), 1 µl de la solution de marqueur (7,5mM dans DMF) a été ajouté. Après incubation à 65°C pendant 30 minutes, chaque produit de réaction a été hybridé, détecté et analysé sur une puce à ADN (Affymetrix, Santa Clara, CA USA) selon le protocole fourni par le constructeur. Ces puces sont conçues pour l'identification de région particulière, et dans le cas présent de la région 213-415 de la séquence M20940 « Genbank » de l'ARN 16S de *Mycobacterium tuberculosis.* Dans le cas de la fluorescéine cadavérine ou de la fluorescéine hydrazide, la séquence a été retrouvée à 66%. Ceci indique que dans chaque cas le marqueur a été introduit pendant la fragmentation générant ainsi des fragments fluorescents et détectables. Une telle identification est bien décrite dans l'article d'A. Troesch et al, J. Clin Microbiol., 37(1), p. 49-55, 1999.

### Exemple 4 - Marquage pendant la fragmentation via un marqueur portant un halogénure de méthyle :

Il est connu qu'un monophosphate peut être substitué par un marqueur portant un groupement phényl-méthyle halogénure (Voir T. Furuta et al., J. Chem. Soc. Perkin Trans. 1 3139- 3142, 1993). La 5-bromo-fluorescéine, représentée à la figure 5, fait partie de cette catégorie de marqueurs halogénés.

Sachant que la réaction de fragmentation libère des extrémités 3'-monophosphate cyclique, en équilibre avec les formes ouvertes, l'étape de fragmentation peut servir pour attacher un marqueur sur les groupements phosphate en 3' des fragments ARN.

### A - Marquage des amplicons ARN obtenus par amplification TMA :

Des amplicons ARN 16S (330 nucléotides) de *Mycobactéries* ont été préparés par amplification TMA, comme décrit dans l'exemple 1-A. La 5-bromo-fluorescéine a été obtenue chez Molecular Probes (Eugene, OR, USA). Le chlorure de manganèse a été obtenu chez Sigma et l'imidazole chez Aldrich.

### A - 1. Marquage de 50 µl de TMA : protocole utilisant 6 mM d'imidazole et 60 mM du chlorure de manganèse :

A 165 µl d'eau RNase free (Sigma), dans un tube en polypropylène de 5 ml, dont les dimensions sont de 12 mm de diamètre pour 75 mm de longueur, on ajoute 15 µl d'une solution d'imidazole (0,1 M), 15 µl d'une solution de chlorure de manganèse (1 M), 2,5 µl la 5-bromofluorescéine (100 mM) dans le DMSO puis 50µl de produits TMA. Le mélange est homogénéisé par agitation au vortex et incubé à 60°C pendant 30 mn.

Après incubation, la solution a été utilisée sans aucune purification pour l'hybridation et la détection sur puce à ADN. (Affymetrix, Santa Clara, CA USA), également appelée biopuce. Le protocole utilisé pour cette étape d'hybridation est celui fourni par le constructeur. Ces puces sont conçues pour l'identification de la région 213-415 de la séquence M20940 «Genbank» de l'ARN 16S de *Mycobacterium tuberculosis.* Les résultats sont donnés dans le tableau 1 ci-dessous :

**Tableau 1 : Résultats du marquage des fragments d'ARN marqués sans purification**

| Score obtenu | Intensité Médiane (Rfu) | Bruit de fond (Rfu) |
|---|---|---|
| 91,2 % | 1727 | 834 |

Le pourcentage d'identification ou score obtenu correspond, dans l'identification par la technologie de la biopuce, à un pourcentage d'analyse par rapport à la séquence de référence. La séquence est identifiée à 91,2% et l'intensité médiane est de 1727 Rfu. Ceci indique que dans chaque cas le marqueur a été introduit pendant la fragmentation générant ainsi des fragments fluorescents et détectables. Il est aussi important de noter que le produit de réaction de marquage a été hybridé directement sur la puce sans aucune purification préalable. Ce résultat est très intéressant et montre que des ARN transcrits non purifiés peuvent être marqués de manière efficace par ce protocole de fragmentation utilisant l'imidazole et le chlorure de manganèse.

Dans un deuxième temps, nous avons tenté le marquage de 50 µl puis 100 µl (volume total) d'une réaction TMA décrite dans l'exemple 1-A, en utilisant le protocole de marquage décrit ci-dessus et une étape de purification avant hybridation sur la puce à ADN.

Après incubation à 65°C, les deux produits de réaction de marquage contenant 50 µl et 100 µl de TMA ont été traités au butanol-1 pour extraire l'excès de la 5-bromofluorescéine. Cette extraction a été réalisée avec deux fois 1ml de butanol-1 saturé en eau. Une telle extraction, comme celles qui suivront, est bien connue de l'état de la technique. Des informations complémentaires peuvent notamment être trouvées dans le document Sambrook, Fritsch & Maniatis, Molecular Cloning (Second Edition, 1.46) Cold Spring Harbor Laboratory Press, 1989. Les résultats sont donnés dans le tableau 2 ci-dessous. Ces mesures sont effectuées après hybridation et lecture sur la biopuce.

**Tableau 2 : Résultats du marquage des fragments d'ARN marqués avec purification**

| Volume de TMA | Score obtenu | Intensité Médiane (Rfu) | Bruit de fond (Rfu) |
|---|---|---|---|
| 50 µl | 95,9 % | 6867 | 169 |
| 100 µl | 96,7% | 5775 | 275 |

Le pourcentage d'identification ou score obtenu est voisin de 100% et les intensités obtenues sont très élevées. Ceci montre qu'une étape de purification avant hybridation peut améliorer les pourcentages d'identification, les intensités de marquage et réduire le bruit de fond. En effet, d'éventuels cycles de lavage post-hybridation supplémentaires ne sont plus nécessaires.

### A - 2. Marquage de 50 µl de TMA : protocole utilisant 30 mM d'imidazole et 30 mM du chlorure de manganèse

A 112 µl d'eau RNase free (Sigma) dans un tube en polypropylène de 5 ml, on ajoute 75 µl d'une solution d'imidazole (0,1 M), 7,5 µl d'une solution de chlorure de manganèse (1 M), 2,5 µl la 5-bromofluorescéine (100 mM) dans le DMSO puis 50 µl de produits TMA. Le mélange est homogénéisé par agitation au vortex et incubé à 60°C pendant 30 mn.

Après incubation, les deux produits de réaction de marquage sur 50 µl de produits TMA ont été traités au butanol-1 pour extraire l'excès de la 5-bromofluorescéine. Ces mesures sont effectuées après hybridation et lecture sur la biopuce. Cette extraction a été réalisée avec deux fois 1 ml de butanol-1 saturé en eau. Les résultats sont donnés dans le tableau 3 ci-dessous.

**Tableau 3 : Résultats du marquage des fragments d'ARN marqués avec purification**

| Volume de TMA | Score obtenu | Intensité Médiane (Rfu) | Bruit de fond (Rfu) |
|---|---|---|---|
| 50 µl | 92,7 % | 444 | 131 |

La séquence amplifiée est identifiée à 92,7% et l'intensité médiane est de 444 Rfu. Ces résultats montrent que le protocole utilisant 30 mM d'imidazole et 30 mM de MnCl₂ produit des fragments d'amplicon marqués. Cependant le niveau d'intensité est inférieur à celui obtenu avec le protocole imidazole/MnCl₂ = 6 mM/60 mM.

Cette stratégie peut être optimisée. La concentration élevée en sel métallique est certainement très importante pour la réaction de marquage. D'autres métaux ou d'autres tampons peuvent être utilisés pour réaliser cette réaction de marquage pendant la fragmentation.

### B - Marquage des ARN transcrits obtenus par transcription post-amplification TMA:

Des cibles ARN transcrits 16S (330 nucléotides) de mycobactéries ont été préparées par transcription, comme décrit dans l'exemple 1-B.

A 165 µl d'eau RNase free (Sigma) dans un tube en polypropylène de 5 ml, on ajoute 15 µl d'une solution d'imidazole (0,1 M), 15 µl d'une solution de chlorure de manganèse (1 M), 2,5 µl la 5-bromofluorescéine (100 mM) dans le DMSO puis 50 µl de produit de transcription. Le mélange est homogénéisé par agitation au vortex et incubé à 60°C pendant 30 mn.

Après incubation, la solution a été traitée comme décrit dans l'exemple 4 (A - 1.) pour éliminer l'excès de la 5-bromofluorescéine. L'hybridation et la détection ont été réalisées ensuite sur la puce à ADN (Affymetrix, Santa Clara, CA USA), conçues pour l'identification de la région 213-415 de la séquence M20940 «Genbank» de l'ARN 16S de *Mycobacterium tuberculosis.* Les résultats sont donnés dans le tableau 4 ci-dessous.

**Tableau 4 : Résultats du marquage des ARN obtenus par transcription post-amplification TMA**

| Score obtenu | Intensité Médiane (Rfu) | Bruit de fond (Rfu) |
|---|---|---|
| 97,6 % | 1426 | 147 |

La séquence est identifiée à 97,6% et l'intensité médiane est de 1426 Rfu. Ce résultat montre que la stratégie de marquage pendant la fragmentation utilisée pour marquer des ARN produits par transcription post-TMA est efficace. Ces cibles ARN transcrits sont utilisées dans la réaction de marquage sans aucune purification.

### C - Marquage des ARN transcrits contenant des thiophosphates post-amplification PCR :

Des cibles ARN 16S (330 nucléotides) de mycobactéries ont été produites par des réactions de transcription post-PCR où 100% de l'adénine tri-phosphate (ATP) a été remplacée par l'ATP-α-thio (deux manipulations ont été réalisées) ou bien 100% de la cytidine tri-phosphate (CTP) a été remplacée par la CTP-α-thio (une manipulation a été réalisée), comme indiqué dans l'exemple 1-D.

Ces ARN ont été marqués en utilisant le protocole décrit ci-dessus en présence de l'imidazole et du chlorure de manganèse. Après incubation à 65°C pendant 30 mn, le produit de réaction a été hybridé, détecté et analysé sur puce à ADN (Affymetrix, Santa Clara, CA USA) selon le protocole fourni par le constructeur. Les résultats sont donnés dans le tableau 5 ci-dessous.

**Tableau 5 : Résultats du marquage des ARN contenant des thiophosphates obtenus par transcription post-amplification PCR**

| Transcrits | Score obtenu | Intensité Médiane (Rfu) |
|---|---|---|
| ATP-α-S | 97,1% 100% | 1186 1290 |
| CTP-α-S | 93,6% | 1027 |

Les séquences sont identifiées à 97,1 et 100% dans le cas de l'ATP-α-S et à 93,6% dans le cas de la CTP-α-S. Les intensités médianes sont comprises entre 1000 et 1300 Rfu. Ce résultat montre que la stratégie de marquage pendant la fragmentation utilisée pour marquer des ARN, contenant des thio-phosphates, est efficace, et même aussi efficace que pour marquer des nucléotides ne contenant pas de soufre. Ces cibles ARN transcrits sont utilisées dans la réaction de marquage sans aucune purification.

### Exemple 5 - Marquage d'ARN naturel :

Une suspension bactérienne est obtenue à partir d'une souche pure isolée sur milieu solide. Cette suspension réalisée en eau stérile est standardisée à un indice de 2 MacFarland (soit environ 3.10⁸ bactéries/ml) puis est centrifugée en tube Eppendorf pendant 1 min.

L'ARN est extrait de la biomasse bactérienne de la façon suivante. Le culot bactérien est lysé par resuspension dans 100 µl de tampon de lyse (Tris.HCl 30 mM, EDTA 5mM, NaCl 100 mM SDS 2%, protéinase K 5 mg /ml, pH 7,3 en présence de 50 µl de billes de verre rondes de 100 µm de diamètre, fabrication VIA1 France). On vortexe pendant 30 sec. Puis on incube pendant 15 mn à 37°C. On ajoute alors 100 µl de phénol saturé. On soumet à un vortex pendant 30 s. La phase aqueuse est récupérée puis extraite avec 100 µl de chloroforme. On soumet à nouveau à un vortex pendant 30 s. La phase aqueuse est de nouveau récupérée. Deux extractions éther sont conduites puis ce solvant est évaporé. Il reste alors environ 50 µl de phase aqueuse contenant les ARN totaux. De manière alternative pour cette extraction on peut utiliser le kit Qiagen RNeasy (marque commerciale).

Le marquage des ARN totaux de la biomasse bactérienne par la technique décrite dans la présente invention est réalisé de la façon suivante par ajout des réactifs dans l'ordre suivant :
- totalité des ARN extraits (50 µl),
- eau (qsp 100 µl),
- 6 µl imidazole 1 M (60 mM final),
- 6 µl MnCl₂ 1 M (60 mM final),
- on soumet la solution à un vortex pendant 10 s,
- 2 µl de 5-Bromo-fluorescéine 50 mM (1 mM final),
- on homogénéise en prélevant plusieurs fois à l'aide d'une pipette,
- on soumet à nouveau à un vortex pendant 1 s,
- on centrifuge 1 s pour collecter en bas du tube, et
- on incube 30 mn à 60 °C.

Pour éliminer le marqueur libre en excès, on procède ensuite de la façon suivante. A partir des 100 µl marqués on ajoute :
- 40 µl d'ADN de sperme de saumon
- 100 µl d'acétate de sodium 3 M (610 mM) final) pH 5,2
- 250 µl d'isopropanol froid (-20°C)

On soumet à un vortex, puis on centrifuge 1 mn. On élimine le surnageant. Le culot est alors remis en suspension dans le tampon d'hybridation (6 x SSPE, 5mM DTAB, betaïne 3 M, Triton 0.05 %, 250 µg/ml d'ADN de sperme de hareng)

On visualise sur un gel d'agarose 1% avec le bromure d'éthidium :
- un aliquote de la préparation d'ARN totaux avant marquage selon l'invention (A),
- un aliquote de la préparation d'ARN totaux après marquage selon l'invention (B).

Après visualisation du bromure d'éthidium sous ultraviolets (UV), on note :
- pour le puits A la présence des bandes caractéristiques d'ARN majoritaires, avec de haut en bas: 23S, 16S puis une bande diffuse correspondant à la population d'ARN messager,
- pour le puits B la présence en bas du gel d'une population correspondant au mélange total d'ARN fragmenté.

Après visualisation de la fluorescéine, on note :
- pour le puits A : rien du tout,
- pour le puits B la présence en bas du gel d'une population correspondant au mélange d'ARN fragmenté et marqué de manière covalente à la fluorescéine ce qui montre que les ARN ont été fragmentés et marqués. Ce contrôle a été effectué pour toutes les espèces bactériennes décrites dans le tableau ci-dessous.

Un aliquote des ARN ainsi marqués est hybridé sur un support solide en verre (biopuce) où sont greffés par photolithographie des oligonucléotides permettant l'identification de ces différentes espèces selon un procédé équivalent à celui décrit dans l'article d'A. Troesch et al, J. Clin Microbiol., 37(1), p. 49-55, 1999. Les résultats sont reproduits sur le tableau 6 suivant.

**Tableau 6 : Résultats du marquage des ARN naturels de différentes espèces**

| **Espèces** | **Référence souchier international** | **Référence souchier interne** | **Score obtenu** |
|---|---|---|---|
| *Escherichia coli* | ATCC11775T | 7308009 | 86,3% |
| *Streptococcus pneumoniae* | NCTC7465T | 7804060 | 87,2% |
| *Klebsiella pneumoniae* | ATCC13883T | 7308012 | 84,6% |
| *Pseudomonas aeruginosa* | ATCC10145 | 7309001 | 79,1% |
| *Enterobacter cloacae* | ATCC13047T | 7308013 | 81% |
| *Streptococcus agalactiae* | ATCC13813 | 7701031 | 89,1% |
| *Klebsiella oxytoca* | ATCC13182T | 9211047 | 85,5% |
| *Citrobacter freundii* | ATCC29935T | 9410068 | 81,5% |
| *Salmonella typhimurium* | --- | 9810059 | 71,3% |

L'espèce ayant le score le plus élevé sur la biopuce est toujours l'espèce recherché, le score obtenu est indiqué sur la colonne de droite.

Pour toutes les espèces, l'identification est correcte, montrant que le procédé de la présente invention est efficace aussi bien sur ARN naturels que sur des ARN issus d'une technique d'amplification.

## Revendications

1. Procédé de marquage d'un acide ribonucléique (ARN) de synthèse ou naturel, **caractérisé en ce qu'**il consiste :
- à fragmenter l'ARN de manière non spécifique par voie chimique, pour générer une pluralité de fragments d'ARN, et
- à marquer chaque fragment dudit ARN au niveau du phosphate terminal ayant été libéré lors de la fragmentation, ledit phosphate terminal étant situé à l'extrémité 3' par fixation sur le phosphate en position 2', en position 3' ou en position 2'-3'monophosphate cyclique, par rapport au ribose, d'une fonction réactive portée par un marqueur ou pouvant s'associer ultérieurement à un marqueur, et
- à purifier le produit de réaction de marquage.

2. Procédé, selon la revendication 1, **caractérisé en ce que** la fragmentation et le marquage s'effectuent en une étape.

3. Procédé, selon la revendication 1, **caractérisé en ce que** la fragmentation et le marquage s'effectuent en deux étapes.

4. Procédé, selon l'une quelconque des revendications 1 à 3, **caractérisé en ce que** la fragmentation et/ou le marquage de l'extrémité 3' d'un fragment d'ARN s'effectue par fixation, sur ledit phosphate terminal, d'une fonction nucléophile, électrophile, halogénure portée par un marqueur ou pouvant s'associer ultérieurement à au moins un marqueur.

5. Procédé, selon l'une quelconque des revendications 1 à 4, **caractérisé en ce que** la fragmentation par voie chimique de l'ARN est réalisée par des cations métalliques associés ou non à un catalyseur chimique.

6. Procédé, selon la revendication 5, **caractérisé en ce que** les cations métalliques sont des ions Mg⁺⁺, Mn⁺⁺, Cu⁺⁺ Co⁺⁺ et/ou Zn⁺⁺, et que le catalyseur chimique est constitué par de l'imidazole, un analogue substitué, par exemple le N-méthyl-imidazole, ou toute molécule chimique ayant une affinité pour l'ARN et portant un noyau imidazole ou un analogue substitué.

7. Procédé, selon l'une quelconque des revendications 1 à 6, **caractérisé en ce que** le marquage de l'extrémité 3'd'un fragment d'ARN s'effectue par fixation, sur ledit phosphate terminal, d'une molécule R-X, où R est constitué par le marqueur et X est l'agent de liaison entre le marqueur et l'ARN, tel qu'un groupement hydroxyle, amine, hydrazine, alcoxylamine, halogénure d'alkyle, phényl-méthyle halogénure, iodoacétamide, maléimide.

## Claims

1. A process for labeling a synthetic or natural ribonucleic acid (RNA), **characterized in that** it consists in:
non specifically and chemically fragmenting the RNA, to produce a plurality of RNA fragments, and
labeling each fragment of said RNA at the level of the terminal phosphate having been freed during the fragmentation, said terminal phosphate being located at the 3' end by linking to the phosphate in 2' position, in 3' position or in cyclic monophosphate 2'-3' position with respect to ribose, of a reactive function linked to a label or being capable of being subsequently linked to at least one label; and
purifying said labeling reaction product.

2. The process according to Claim 1, **characterized in that** the fragmenting and the labeling are effected in one step.

3. The process according to Claim 1, **characterized in that** the fragmenting and the labeling are effected in two steps.

4. The process according to any one of Claims 1 to 3, **characterized in that** the fragmenting and/or the labeling on the 3' end of an RNA fragment, is effected by binding on said terminal phosphate, a nucleophilic, electrophilic or halide function being linked to a label or being capable of being subsequently linked to at least one label.

5. The process according to any one of Claims 1 to 4, **characterized in that** the chemical fragmentation of RNA is carried out by means of metal cations, said metal cations being combined with a chemical catalyst or not.

6. The process according to Claim 5, **characterized in that** the metal cations are Mg⁺⁺, Mn⁺⁺, Cu⁺⁺, Co⁺⁺ and/or Zn⁺⁺ ions, and the chemical catalyst consists of imidazole, a substituted analogue, for example N-methylimidazole, or any chemical molecule which has an affinity for the RNA and which carries an imidazole nucleus or a substituted analogue.

7. Process according to any one of Claims 1 to 6, **characterized in that** the labeling of the 3' end of an RNA fragment is effected by binding a molecule R-X, where R consists of the label and X is the agent for binding the label to the RNA, such as a hydroxyl, amine, hydrazine, alkoxylamine, alkyl halide, phenylmethyl halide, iodoacetamide and maleimide groups, to said terminal phosphate.

## Patentansprüche

1. Verfahren zur Markierung einer synthetischen oder natürlichen Ribonukleinsäure (RNA), **dadurch gekennzeichnet, daß** es aus den folgenden Schritten besteht:
- unspezifische chemische Fragmentierung der RNA, um eine Mehrzahl von RNA-Fragmenten zu erzeugen, und
- Markieren jedes Fragments dieser RNA am endständigen Phosphat, das bei der Fragmentierung freigesetzt worden ist, wobei sich diese s endständige Phosphat am 3' -Ende befindet, und zwar durch Anbringen einer reaktionsfähigen Funktion, die von einem Marker getragen wird oder die sich später an einen Marker anlagern kann, an das Phosphat in 2' -Stellung, in 3' -Stellung oder 2' - 3'-Stellung des cyclischen Monophosphats in bezug auf die Ribose, und
- Aufreinigung des Produkts der Markierungsreaktion.

2. Verfahren nach Anspruch 1, **dadurch gekennzeichnet, daß** die Fragmentierung und die Markierung in einem Schritt vorgenommen werden.

3. Verfahren nach Anspruch 1, **dadurch gekennzeichnet, daß** die Fragmentierung und die Markierung in zwei Schritten vorgenommen werden.

4. Verfahren nach einem der Ansprüche 1 bis 3,
**dadurch gekennzeichnet, daß** die Fragmentierung und/oder die Markierung des 3' -Endes eines RNA -Fragments durch Anbringen einer nukleophilen, elektrophilen Halogenidfunktion, die von ein em Marker getragen wird oder die sich später an mindestens einen Marker anlagern kann, an das genannte endständige Phosphat.

5. Verfahren nach einem der Ansprüche 1 bis 4,
**dadurch gekennzeichnet, daß** die chemische Fragmentierung der RNA mit Hilfe von Met allkationen, die gegebenenfalls an einen chemischen Katalysator angelagert sind, erfolgt.

6. Verfahren nach Anspruch 5, **dadurch gekennzeichnet, daß** es sich bei den Metallkationen um Mg ⁺⁺-, Mn ⁺⁺-Cu⁺⁺-, Co ⁺⁺- und/oder Zn ⁺⁺-Ionen handelt, und daß der chemische Katalysator aus Imidazol, einem substituierten Analog, zum Beispiel N -Methylimidazol, oder einem beliebigen chemischen Molekül mit Affinität für die RNA, das einen Imidazolkern oder ein substituiertes Analog trägt, besteht.

7. Verfahren nach einem d er Ansprüche 1 bis 6,
**dadurch gekennzeichnet, daß** die Markierung des 3' -Endes eines RNA-Fragments durch Anbringen eines Moleküls R -X an diesem endständigen Phosphat erfolgt, wobei R aus dem Marker besteht und X das Bindeglied zwischen dem Marker und der RN A bedeutet, wie zum Beispiel eine Hydroxyl-, Amin -, Hydrazin -, Alkoxylamin -, Alkylhalogenid-, Phenylmethylhalogenid -, Ioda cetamid- oder Maleimidgruppe.
